Europäisches Patentamt

European Patent Office

Office européen des brevets

(19)

(11) EP 0 478 620 B1

(12) **EUROPEAN PATENT SPECIFICATION**

(45) Date of publication and mention
of the grant of the patent:
**11.03.1998 Bulletin 1998/11**

(21) Application number: **90909175.3**

(22) Date of filing: **12.06.1990**

(51) Int Cl.6: **G01N 33/72**, G01N 33/68

(86) International application number:
**PCT/GB90/00907**

(87) International publication number:
**WO 90/15995 (27.12.1990 Gazette 1990/29)**

(54) **METHOD AND APPARATUS FOR THE DETERMINATION OF GLYCOSYLATED PROTEIN**

VERFAHREN UND VORRICHTUNG ZUR BESTIMMUNG VON GLYKOSYLIERTEM PROTEIN

PROCEDE ET APPAREIL DE DETERMINATION D'UNE PROTEINE GLYCOSYLEE

(84) Designated Contracting States:
**AT BE CH DE DK ES FR GB IT LI LU NL SE**

(30) Priority: **13.06.1989 GB 8913586**

(43) Date of publication of application:
**08.04.1992 Bulletin 1992/15**

(73) Proprietor: **CLINICAL INNOVATIONS LIMITED
Coventry CV4 7EZ (GB)**

(72) Inventors:
• **WALLWORTH, Denise Maria
Congleton CW12 3UA (GB)**
• **GREEN, Bryan
Coventry CV3 5LS (GB)**

(74) Representative: **Low, Peter John et al
Wilson, Gunn, M'Caw,
41-51 Royal Exchange,
Cross Street
Manchester, M2 7BD (GB)**

(56) References cited:
**DE-A- 3 720 736          FR-A- 2 464 475**

• **E. Lester (1989) Ann. Clin. Biochem. 26:213-219**

Remarks:
The file contains technical information submitted after the application was filed and not included in this specification

## Description

TECHNICAL FIELD

This invention relates to a method of determing the glycosylated protein level of a sample and an apparatus for use in the method and is particularly, but not exclusively, relevant to determining the level of glycosylated haemoglobin and other glycosylated proteins in biological fluids.

BACKGROUND ART

Known methods of determining the concentration of glucose in blood at a particular instant include a range of stick tests and meters which are often used for routine assessment by a patient, for example a diabetic.

However, the results provided by such methods and apparatus cannot be relied upon to give an accurate retrorespective indication of the long term control of blood glucose concentration.

When the patient visits his doctor a sample of blood is taken from the patient which is forwarded to a laboratory for analysis. Laboratory analysis involves the determination of glycosylated haemoglobin or other glycosylated protein which provides a reliable retrorespective indication of the long term averaged glucose concentration of the blood.

Techniques which are currently available for laboratory analysis include affinity gel chromatography and ion exchange liquid chromatography (IELC).

The time taken to analyse a blood sample using affinity chromatography is in the order of two to three hours.

The IELC apparatus is bulky and expensive. Also the time taken to prepare a sample prior to running a chromatogram using IELC is in the order of forty minutes. The time taken to run the chromatogram is in the order of seven minutes.

None of these methods and apparatus is suitable for use by the medical practioner in a clinic.

French Patent Specification No.2464475 described a method of affinity for determining the level of glycosylated haemoglobin in blood. But that method also does not give a result sufficiently quickly.

In practice the results of these tests do not return from the laboratory for several days and the doctor cannot inform the patient of the results of this test and advise an appropriate change of diet and/or therapy if required until these results have arrived.

The present invention has been made from a consideration of this problem.

According to a first aspect of the invention there is provided a method of determining the glycosylated protein level of a sample comprising bringing a quantity of sample into contact with a material having an affinity for the glycosylated protein whose level is to be determined and optically, electrochemically, or enthalpiometrically examining the sample characterised in that the sample and/or the material is examined before contact with the material and/or sample respectively and in that the sample and/or material is examined after contact with the material and/or sample respectively to obtain a substantially immediate reading or readings indicative of the glycosylated protein level in the sample.

According to a second aspect of the present invention there is provided an apparatus for determining the glycosylated protein level of a sample by the method defined above, comprising a support for a material having an affinity for the glycosylated protein whose level is to be determined, means for bringing the sample into contact with said material characterised by means for optically, electrochemically or enthalpiometrically examining the sample and/or optically, electrochemically, or enthalpiometrically examining said material before contact of the sample with the material and for optically, electrochemically or enthalpiometrically examining the sample and/or optically, electrochemically, or enthalpiometrically examining said material after contact of the sample with the material.

The material having an affinity for the glycosylated protein is preferably phenylboronic acid or a derivative thereof.

The said material is preferably bonded to a suitable support such as a hydroxyethyl methacrylate copolymer in microparticulate form or a nylon membrane. In a further alternative the said material may be present as a film.

The level of any glycosylated protein may be determined such as glycosylated haemoglobin, glycosylated albumin or glycosylated serum protein.

The sample is preferably taken using a wick which is chemically treated with a lysing agent such as synperonic N. At least one indication is provided on the wick in order to indicate the correct sample size. This indication is provided part way up the wick in order that the wick has additional capacity to absorb further sample. At least a part of the wick which is located above the wick may be covered preferably by a transparent or translucent cover, such that when the wick is brought into contact with a reaction mixture so as to transfer the sample into the reaction mixture the amount of dissolution from that covered part of the wick is reduced. The diameter of the top of the wick is preferably less than the diameter of the body of the wick. The wick is preferably made from a cellulose acetate material or a derivative thereof. This cellulose acetate material is similar to that conventionally used in cigarette filter tips.

Preferably the sample is diluted in buffer solution prior to administering the sample to the said material.

Preferably the said material is maintained in a container or cartridge through the wall of which one or more apertures pass so as to allow the sample to gain access to the said material.

The amount of sample used is preferably small and in any event much smaller than samples required for known methods.

In a preferred embodiment of the invention the sample may be added to a container containing said material such as coupled phenylboronic acid. Monitoring means such as a photometer may be used to optically determine the total quantity of a certain protein prior to adding the sample to the cartridge (READ 1). Monitoring means may be used to optically determine the total quantity of the protein less the total quantity of any glycosylated protein (READ 2) after the sample has passed through the cartridge.

The percentage of that glycosylated protein (% GP) in the sample may then be derived from the following equation:

$$\%GP = \frac{READ\ 1 - READ\ 2}{READ\ 1} \times 100 \quad \text{Equation A}$$

Alternatively the sample may be held in a container to which a cartridge of the said material such as coupled phenylboronic acid is added. A monitoring means such as a photometer may be used to determine the total quantity of a certain protein (% GP) prior to adding the cartridge to the sample (READ 1). A monitoring means may then be used to optically determine the total quantity of protein in the sample less the total quantity of glycosylated protein (READ 2) once the cartridge has been added to the sample.

The percentage of the glycosylated protein (% GP) in the sample may then be determined using the above Equation A.

In a further alternative the cartridge of said material may comprise coupled phenylboronic acid which may have a certain optical quality such as reflectance, retroreflectance, fluroscence or phosphorescence which varies once the analytes, for example glycosylated protein is bonded thereto. This change may be monitored so as to determine the quantity of glycosylated protein present in the sample.

If reflectance is monitored then the percentage of glycosylated protein (% GP) in the sample may be derived from the following equation:

$$\%GP = \frac{READ\ 2}{READ\ 1} \times 100 \quad \text{Equation B}$$

where READ 2 is the reflectance of the material in the cartridge after the sample has been brought into contact with the material and READ 1 is the absorbence of the sample prior to bringing the sample into contact with the material.

BRIEF DESCRIPTION OF THE DRAWINGS

In order that the invention may be more readily understood specific embodiments thereof will now be described by way of example only with reference to the accompanying drawings in which:-

Fig. 1 is a preferred form of metering device for taking a blood sample the glycosylated protein level of which is to be determined;

Fig. 2 is a side elevation of one embodiment of the apparatus of the present invention;

Fig. 3 is a side elevation of a second embodiment of the apparatus of the present invention; and

Fig. 4 is a side elevation of the apparatus of Fig.3 in which the said material has been added to the sample.

BEST MODES FOR CARRYING OUT THE INVENTION

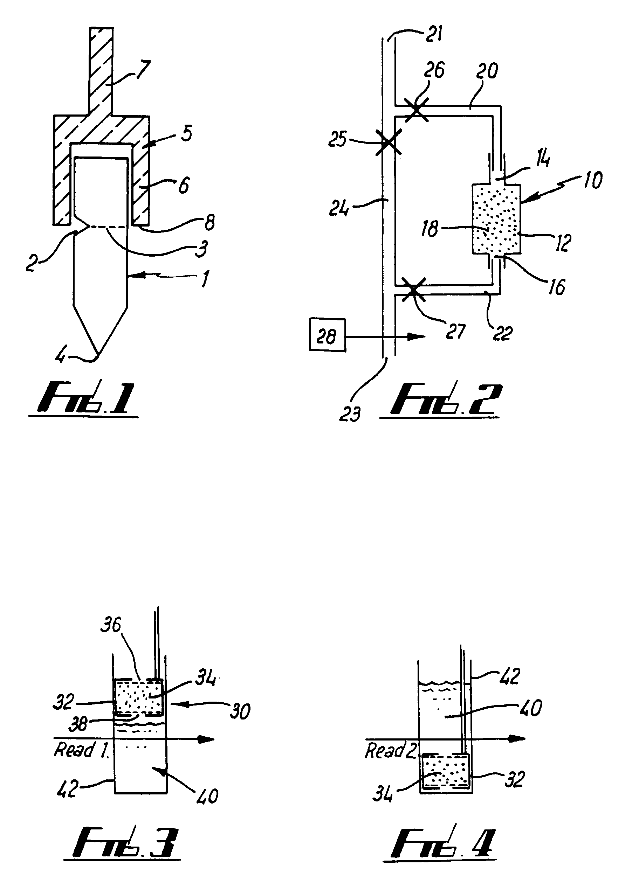

Referring to Fig. 1 there is shown a wick 1 comprising a pad made from a cellulose acetate based material. This material allows in the order of 60-70% dissolution of blood samples. An indentation 2 and marking 3 are provided part way up the wick 1 so as to provide an indication to the user of the required quantity of sample to be taken up by the wick 1. It is noted that the upper part of the wick 1 is not saturated by the sample when the required size of sample has been taken.

The wick 1 is chemically treated with a lysing agent such as synperonic N. This may be achieved by bringing the wick 1 into contact with the lysing agent until the wick 1 is saturated with lysing agent. The wick 1 is subsequently allowed to dry.

The tip 4 of the wick 1 is seen to have a more narrow diameter than the body of the wick 1. Alternatively the diameter of the wick 1 may be uniform.

The top of the wick 1 is located in cap 5. The cap 5 comprises a cover 6 and a handle 7. The height of the cover 5 is such that when in position the base 8 of the cover 5 is substantially adjacent indications 2, 3. The cover 5 is preferably made from transparent or translucent plastics material in order that the upper part of the wick 1 can be seen through the cover 5.

When in use the tip 4 of the wick 1 is brought into contact with the blood causing blood to rise up the wick by capillary action. As the blood rises up the wick 1 the lysing agent located on the wick 1 causes rupture of the erythrocyte membrane allowing release of the hameoglobin from the cells.

As the blood level reaches the indications 2, 3 the wick 1 is removed from the sample. The resultant residual amount of blood located at the tip 4 of the wick 1 causes the blood level to rise above the indications 2, 3 resulting in an oversampling. This will lead to a small percentage error. However more importantly as the lysing agent is present along the entire length of the wick substantially no non-lysed erythrocytes will be present on the wick. This is a very important consideration with this type of analysis.

The sample is transferred from the wick 1 by shaking the wick 1 in buffer solution causing dissolution of the sample. However as the cap 5 covers the upper part of the wick 1 dissolution of any sample above the indications 2, 3 is substantially prevented.

Referring to Fig. 2 an apparatus 10 for determining the glycosylated protein level of the sample such as in this example the determination of the glycosylated haemoglobin level of blood comprises a cartridge 12 with an inlet 14 and outlet 16. The cartridge contains coupled phenylboronic acid adsorbed to a support 18.

One end of an entrance tube 20 is received in the inlet 14 and the other end of the entrance tube 20 is connected to a bypass tube 24. One end of a waste tube 22 is received in the exit 16 and the other end of the waste tube 22 is connected to the bypass tube 24.

A tap 25 is provided in the bypass tube 24 intermediate the mouths of the entrance tube 20 and waste tube 22.

A further tap 26 is provided at the mouth of the entrance tube 20 and a tap 27 is provided at the exit of the waste tube 22.

In use, for example, a blood sample is diluted in a buffer solution and is added to one end 21 of the bypass tube 24. With taps 26 and 27 closed and tap 25 open this mixture is urged down the bypass tube 24. A photometer 28 is used to determine the total level of haemoglobin in the sample (READ 1) at the exit 23 of the bypass tube 24.

Subsequently tap 25 is closed and taps 26, 27 are opened and the mixture is urged down the entrance tube 20 and over the phenylboronic acid and its support 18 under pressure by a suitable device which is not illustrated.

As the sample passes over the phenylboronic acid the glycosylated haemoglobin in the blood sample is retained in the cartridge 12.

The remainder of the blood sample passes through the cartridge 12, through the waste pipe 22 and the exit 23 of the bypass tube 24.

The photometer 28 is used to determine the level of haemoglobin in the sample less the content of glycosylated haemoglobin (READ 2). The wavelength of light emitted from the photometer may be varied depending on the material in respect of which the measurements are being taken.

In this example the wavelength of emitted light of the photometer is 414nm.

The percentage level of glycosylated haemoglobin in the blood sample may be determined using Equation A.

The time taken to take the necessary measurements is in the order of two minutes.

The cartridge device described is cost effective to produce and may be disposed of once used.

Referring now to Figs. 3 and 4 an apparatus 30 for determining the level of glycosylated protein of a sample for example the glycosylated haemoglobin level of a blood sample comprises a cartridge 32 containing phenylboronic acid coupled to a support such as a co-polymer of hydroxyethylmethacrylate 34. The cartridge 32 has two ports 36, 38.

A blood sample 40 diluted in buffer solution is contained in a vessel 42.

In use a monitoring device such as a photometer is used to optically determine the amount of haemoglobin present in the blood sample 40 (READ 1).

Subsequently the cartridge 32 is entirely immersed in the blood sample 40 and buffer as is shown in Fig. 3 such that the blood sample can gain access to coupled phenylboronic acid via the port 38. Glycosylated haemoglobin in the blood sample 40 has an affinity for the phenylbornoic acid contained in the cartridge and is retained in the cartridge.

A second measurement is then taken using suitable monitoring means such as the photometer to optically determine the total amount of haemoglobin in the blood sample less than the amount of glycosylated haemoglobin (READ 2).

The level of glycosylated haemoglobin in the blood sample may be determined using Equation A.

It can be seen from the above described embodiments that the invention provides a quick, cost effective method of monitoring the level of glycosylated proteins in samples of materials such as blood which may easily be carried out in a doctor's surgery.

It is to be understood that the above described embodiments are by way of illustration only and that many modifications and variations can be made as defined in the claims. For example the level of glycosylated protein in the sample may be determined electrochemically or enthalpiometrically.

## Claims

1. A method of determining the glycosylated protein level of a sample comprising bringing a quantity of sample into contact with a material having an affinity for the glycosylated protein whose level is to be determined and optically, electrochemically, or enthalpiometrically examining the sample characterised in that the sample and/or the material is examined before contact with the material and/or sample respectively and in that the sample and/or material is examined after contact with the material and/or sample respectively to obtain a substantially immediate reading or readings indicative of the glycosylated protein level in the sample.

2. A method of determining the glycosylated protein level of a sample as claimed in Claim 1, wherein said material comprises phenylboronic acid or a derivative thereof.

3. A method of determining the glycosylated protein level of a sample as claimed in Claim 1, wherein said material is bonded to a support.

4. A method of determining the glycosylated protein level of a sample as claimed in claim 3, wherein the support comprises at least one of a hydroxyethyl methacrylate co-polymer in particulate form or a nylon membrane.

5. A method of determining the glycosylated protein level of a sample as claimed in claim 1 or claim 2, wherein said material is present as a film.

6. A method of determining the glycosylated protein level of a sample as claimed in any preceding claim, wherein the sample is diluted in buffer solution prior to administering the sample to the said material.

7. A method of determining the glycosylated protein level of a sample as claimed in any preceding claim, wherein said material is maintained in a container or cartridge.

8. A method of determining the glycosylated protein level of a sample as claimed in any preceding claim, wherein said material comprises any of the following properties either alone or in combination which vary once the glycosylated protein is bonded to said material; reflectance, retroreflectance, fluorescence or phosphorescence.

9. A method of determining the glycosylated protein level of a sample as claimed in claim 7, wherein the container or cartridge is submerged in a quantity of the sample.

10. A method of determining the glycosylated protein level of a sample as claimed in claim 7, wherein the sample is urged through the container or cartridge.

11. A method of determining the glycosylated protein level of a sample as claimed in any preceding claim, wherein the sample comprises at least one of a glycosylated albumin or a glycosylated haemoglobin or a glycosylated serum protein.

12. A method of determining the glycosylated protein level of a sample, as claimed in any preceding claim, wherein the sample is taken by a metering device.

13. A method of determining the glycosylated protein level of a sample as claimed in claim 12, wherein the metering device comprises cellulose acetate or a derivative thereof.

14. A method of determining the glycosylated protein level of a sample as claimed in claim 12 or claim 13, wherein the metering device comprises an indication in order to signify when a certain amount of sample has been taken up by the metering device.

15. A method of determining the glycosylated protein level of a sample in any of claims 12 to 14, wherein the metering device is not completely saturated with the sample when the required amount of sample has been taken.

16. A method of determining the glycosylated protein level of a sample as claimed in any of claims 12 to 15, wherein the metering device comprises a lysing agent.

17. A method of determining the glycosylated protein level of a sample as claimed in any of claims 12 to 16, wherein the tip of the metering device which in use is brought into contact with the substance from which a sample is to be taken is of reduced diameter in relation to the body of the metering device.

18. A method of determining the glycosylated protein level of a sample as claimed in any of claims 12 to 17, wherein the metering device comprises a cap which covers at least a part of the metering device and restrains or prevents transfer of sample from at least part of the metering device.

19. A method of determining the glycosylated protein level of a sample as claimed in any preceding claim, wherein the metering device comprises a wick.

20. An apparatus for determining the glycosylated protein level of a sample by the method according to Claim 1, comprising a support for a material having an affinity for the glycosylated protein whose level is to be determined, means for bringing the sample into contact with said material characterised by means for optically electrochemically or enthalpiometrically examining the sample and/or optically, electrochemically, or enthalpiometrically examining said material before contact of the sample with the material and optically, electrochemically or enthalpiometrically examining the sample and/or optically, electrochemically, or enthalpiometrically examining said material after contact of the sample with the material.

21. An apparatus for determining the glycosylated protein level of a sample as claimed in claim 20, wherein said material comprises phenylboronic acid or a derivative thereof.

22. An apparatus for determining the glycosylated protein level of a sample as claimed in claim 20 or claim 21, wherein said material is bonded to a support.

23. An apparatus for determining the glycosylated protein level of a sample as claimed in claim 22, wherein the support comprises at least one of a hydroxyethyl methacrylate co-polymer in particulate form or a nylon membrane.

24. An apparatus for determining the glycosylated protein level of a sample as claimed in claim 11 or claim 21, wherein said material is present as a film.

25. An apparatus for determining the glycosylated protein level of a sample as claimed in any of claims 20 to 24, wherein the sample is diluted in buffer solution prior to administering the sample to the said material.

26. An apparatus for determining the glycosylated protein level of a sample as claimed in any of claims 20 to 25, wherein said material is maintained in a container or cartridge.

27. An apparatus for determining the glycosylated protein level of a sample as claimed in any of claims 20 to 26, wherein said material comprises any of the following properties either alone or in combination which vary once the glycosylated protein is bonded to said material; reflectance, retroreflectance, fluorescence or phosphorescence.

28. An apparatus for determining the glycosylated protein level of a sample as claimed in claim 26, wherein the container of cartridge is submerged in a quantity of the sample.

29. An apparatus for determining the glycosylated protein level of a sample as claimed in claim 26, wherein the sample is urged through the container or cartridge.

**Patentansprüche**

1. Verfahren zum Bestimmen des Gehalts einer Probe an glycosyliertem Protein, welches umfaßt:
Inkontaktbringen einer Menge der Probe mit einem Material, das eine Affinität für das glycosylierte Protein besitzt, dessen Gehalt bestimmt werden soll, und Untersuchen der Probe auf optischem, elektrochemischem oder enthalpiometrischem Wege, dadurch **gekennzeichnet**,
daß die Probe und/oder das Material vor dem Kontakt mit dem Material und/oder der Probe untersucht wird, und dadurch, daß die Probe und/oder das Material nach dem Kontakt mit dem Material und/oder der Probe untersucht wird, um im wesentlichen ein sofortiges Ergebnis oder sofortige Ergebnisse zu erhalten, die Rückschlüsse auf den Gehalt an glycosyliertem Protein in der Probe erlauben.

2. Verfahren zum Bestimmen des Gehalts einer Probe an glycosyliertem Protein gemäß Anspruch 1, worin das Material Phenylboronsäure oder ein Derivat derselben umfaßt.

3. Verfahren zum Bestimmen des Gehalts einer Probe an glycosyliertem Protein gemäß Anspruch 1, worin das Material an einen Träger gebunden ist.

4. Verfahren zum Bestimmen des Gehalts einer Probe an glycosyliertem Protein gemäß Anspruch 3, worin der Träger wenigstens eine der Verbindungen eines Hydroxyethyl-methacrylat-Copolymers in zerkleinerter Form oder eine Nylon-Membran umfaßt.

5. Verfahren zum Bestimmen des Gehalts einer Probe an glycosyliertem Protein gemäß Anspruch 1 oder Anspruch 2,
worin das Material als eine Folie vorliegt.

6. Verfahren zum Bestimmen des Gehalts einer Probe an glycosyliertem Protein gemäß irgendeinem der vorhergehenden Ansprüche,
worin die Probe vor der Zugabe der Probe zu dem Material in einer Pufferlösung verdünnt wird.

7. Verfahren zum Bestimmen des Gehalts einer Probe an glycosyliertem Protein gemäß irgendeinem der vorhergehenden Ansprüche,
worin das Material in einem Behälter oder einer Kartusche aufbewahrt wird.

8. Verfahren zum Bestimmen des Gehalts einer Probe an glycosyliertem Protein gemäß irgendeinem der vorhergehenden Ansprüche,
worin das Material entweder allein oder in Kombination eine der folgenden Eigenschaften umfaßt, welche sich ändern, wenn das glycosylierte Protein an das Material gebunden ist: reflektierende Eigenschaft, retroreflektierende Eigenschaft, Fluoreszenz oder Phosphoreszenz.

9. Verfahren zum Bestimmen des Gehalts einer Probe an glycosyliertem Protein gemäß Anspruch 7, worin der Behälter oder die Kartusche in eine Menge der Probe eingetaucht ist.

10. Verfahren zum Bestimmen des Gehalts einer Probe an glycosyliertem Protein gemäß Anspruch 1, worin die Probe durch den Behälter oder die Kartusche gepreßt wird.

11. Verfahren zum Bestimmen des Gehalts einer Probe an glycosyliertem Protein gemäß irgendeinem der vorhergehenden Ansprüche,
worin die Probe wenigstens eine der Verbindungen eines glycosylierten Albumins oder eines glycosylierten Hämoglobins oder eines glycosylierten Serumproteins umfaßt.

**12.** Verfahren zum Bestimmen des Gehalts einer Probe an glycosyliertem Protein gemäß irgendeinem der vorhergehenden Ansprüche, worin die Probe mit einer Dosiervorrichtung entnommen wird.

**13.** Verfahren zum Bestimmen des Gehalts einer Probe an glycosyliertem Protein gemäß Anspruch 12, worin die Dosiervorrichtung Celluloseacetat oder ein Derivat derselben umfaßt.

**14.** Verfahren zum Bestimmen des Gehalts einer Probe an glycosyliertem Protein gemäß Anspruch 12 oder Anspruch 13, worin die Dosiervorrichtung eine Anzeige umfaßt, um anzuzeigen, wenn eine bestimmte Menge der Probe von der Dosiervorrichtung aufgenommen worden ist.

**15.** Verfahren zum Bestimmen des Gehalts einer Probe an glycosyliertem Protein gemäß irgendeinem der Ansprüche 12 bis 14, worin die Dosiervorrichtung nicht vollständig mit der Probe gesättigt ist, wenn die erforderliche Menge der Probe aufgenommen worden ist.

**16.** Verfahren zum Bestimmen des Gehalts einer Probe an glycosyliertem Protein gemäß irgendeinem der Ansprüche 12 bis 15, worin die Dosiervorrichtung ein Lyse-Mittel umfaßt.

**17.** Verfahren zum Bestimmen des Gehalts einer Probe an glycosyliertem Protein gemäß irgendeinem der Ansprüche 12 bis 16, worin die Spitze der Dosiervorrichtung, welche im Gebrauch mit der Verbindung, von der eine Probe genommen werden soll, in Kontakt gebracht wird, verglichen mit dem Körper der Dosiervorrichtung einen reduzierten Durchmesser aufweist.

**18.** Verfahren zum Bestimmen des Gehalts einer Probe an glycosyliertem Protein gemäß irgendeinem der Ansprüche 12 bis 17, worin die Dosiervorrichtung eine Kappe umfaßt, welche wenigstens einen Teil der Dosiervorrichtung bedeckt und den Transfer der Probe von wenigstens einem Teil der Dosiervorrichtung behindert oder unterbindet.

**19.** Verfahren zum Bestimmen des Gehalts einer Probe an glycosyliertem Protein gemäß irgendeinem der vorhergehenden Ansprüche, worin die Dosiervorrichtung einen Docht umfaßt.

**20.** Vorrichtung zum Bestimmen des Gehalts einer Probe an glycosyliertem Protein mit dem Verfahren gemäß Anspruch 1, welche umfaßt: einen Träger für ein Material mit einer Affinität für das glycosylierte Protein, dessen Gehalt bestimmt werden soll, Mittel, um die Probe mit diesem Material in Kontakt zu bringen, gekennzeichnet durch Mittel zum optischen, elektrochemischen oder enthalpiometrischen Untersuchen der Probe und/oder optischen, elektrochemischen oder enthalpiometrischen Untersuchen des Materials vor dem Kontakt der Probe mit dem Material und optischen, elektrochemischen oder enthalpiometrischen Untersuchen der Probe und/oder optischen, elektrochemischen oder enthalpiometrischen Untersuchen des Materials nach dem Kontakt der Probe mit dem Material.

**21.** Vorrichtung zum Bestimmen des Gehalts einer Probe an glycosyliertem Protein gemäß Anspruch 20, worin das Material Phenylboronsäure oder ein Derivat derselben umfaßt.

**22.** Vorrichtung zum Bestimmen des Gehalts einer Probe an glycosyliertem Protein gemäß Anspruch 20 oder Anspruch 21, worin das Material an einen Träger gebunden ist.

**23.** Vorrichtung zum Bestimmen des Gehalts einer Probe an glycosyliertem Protein gemäß Anspruch 22, worin der Träger wenigstens eine Verbindung eines Hydroxyethyl-methacrylat-Copolymers in zerkleinerter Form oder eine Nylon-Membran umfaßt.

**24.** Vorrichtung zum Bestimmen des Gehalts einer Probe an glycosyliertem Protein gemäß Anspruch 11 oder Anspruch 21, worin das Material als Folie vorliegt.

**25.** Vorrichtung zum Bestimmen des Gehalts einer Probe an glycosyliertem Protein gemäß einem der Ansprüche 20 bis 24, worin die Probe vor dem Zugeben der Probe zu dem Material in einer Pufferlösung verdünnt ist.

**26.** Vorrichtung zum Bestimmen des Gehalts einer Probe an glycosyliertem Protein gemäß irgendeinem der Ansprüche 20 bis 25, worin das Material in einem Behälter oder einer Kartusche aufbewahrt wird.

**27.** Vorrichtung zum Bestimmen des Gehalts einer Probe an glycosyliertem Protein gemäß irgendeinem der Ansprüche 20 bis 26, worin das Material entweder allein oder in Kombination eine der folgenden Eigenschaften umfaßt, welche sich ändern, wenn das glycosylierte Protein an das Material gebunden ist: reflektierende Eigenschaft, retroreflektierende Eigenschaft, Fluoreszenz oder Phosphoreszenz.

**28.** Vorrichtung zum Bestimmen des Gehalts einer Probe an glycosyliertem Protein gemäß Anspruch 26, worin der Behälter oder die Kartusche in eine Menge der Probe eingetaucht ist.

29. Vorrichtung zum Bestimmen des Gehalts einer Probe an glycosyliertem Protein gemäß Anspruch 26, worin die Probe durch den Behälter oder die Kartusche gepreßt wird.

**Revendications**

1. Procédé pour déterminer la teneur d'un échantillon en protéine glycosilée, comprenant le fait d'amener une quantité de l'échantillon en contact avec un matériau ayant une affinité pour la protéine glycosilée dont la teneur est à déterminer, et à examiner l'échantillon optiquement, électrochimiquement ou de manière enthalpiométrique, caractérisé en ce que l'échantillon et/ou le matériau est examiné avant son contact avec le matériau et/ou l'échantillon respectivement et en ce que l'échantillon et/ou le matériau est examiné après son contact avec le matériau et/ou l'échantillon respectivement pour obtenir une lecture ou des lectures sensiblement immédiates indications de la teneur de protéine glycosilée dans l'échantillon.

2. Procédé selon la revendication 1, pour déterminer la teneur d'un échantillon en protéine glycosilée, dans lequel ledit matériau comprend de l'acide phénylboronique ou un de ses dérivés.

3. Procédé selon la revendication 1, pour déterminer la teneur d'un échantillon en protéine glycosilée, dans lequel ledit matériau est lié à un support.

4. Procédé selon la revendication 3, pour déterminer la teneur d'un échantillon en protéine glycosilée, dans lequel le support comprend au moins un copolymére d'hydroxyéthyl méthacrylate sous forme particulaire ou une membrane en nylon.

5. Procédé selon la revendication 1 ou la revendication 2, pour déterminer la teneur d'un échantillon en protéine glycosilée, dans lequel ledit matériau se présente sous la forme d'un film.

6. Procédé selon l'une quelconque des revendications précédentes, pour déterminer la teneur d'un échantillon en protéine glycosilée dans lequel l'échantillon est dilué dans une solution tampon avant la mise en présence de l'échantillon et dudit matériau.

7. Procédé selon l'une quelconque des revendications précédentes pour déterminer la teneur d'un échantillon en protéine glycosilée, dans lequel ledit matériau est maintenu dans un conteneur ou une cartouche.

8. Procédé selon l'une quelconque des revendications précédentes, pour déterminer la teneur d'un échantillon en protéine glycosilée, dans lequel ledit matériau comporte, soit seule soit en combinaison, l'une quelconque des propriétés suivantes qui varient lorsque la protéine glycosilée est liée audit matériau : facteur de réflexion, facteur de rétro-réflexion, fluorescence ou phosphorescence.

9. Procédé selon la revendication 7, pour déterminer la teneur d'un échantillon en protéine glycosilée, dans lequel le conteneur ou la cartouche est immergé dans une certaine quantité d'échantillon.

10. Procédé selon la revendication 7, pour déterminer la teneur d'un échantillon en protéine glycosilée, dans lequel on fait passer à force l'échantillon à travers le conteneur ou la cartouche.

11. Procédé selon l'une quelconque des revendications précédentes, pour déterminer la teneur d'un échantillon en protéine glycosilée, dans lequel l'échantillon comprend au moins une albumine glycosilée ou une hémoglobine glycosilée ou une séro-protéine glycosilée.

12. Procédé selon l'une quelconque des revendications précédentes, pour déterminer la teneur d'un échantillon en protéine glycosilée dans lequel l'échantillon est prélevé par un dispositif de mesure.

13. Procédé selon la revendication 12, pour déterminer la teneur d'un échantillon en protéine glycosilée, dans lequel le dispositif de mesure comprend de l'acétate de cellulose ou un de ses dérivés.

14. Procédé selon la revendication 12 ou la revendication 13, pour déterminer la teneur d'un échantillon en protéine glycosilée, dans lequel le dispositif de mesure comprend une indication pour indiquer lorsqu'une certaine quantité d'échantillon a été prélevée par le dispositif de mesure.

15. Procédé selon l'une quelconque des revendications 12 à 14, pour déterminer la teneur d'un échantillon en protéine glycosilée, dans lequel le dispositif de mesure n'est pas complètement saturé par l'échantillon lorsque la quantité nécessaire d'échantillon a été prélevée.

16. Procédé selon l'une quelconque des revendications 12 à 15, pour déterminer la teneur d'un échantillon en protéine glycosilée, dans lequel le dispositif de mesure comprend un agent lysant.

17. Procédé selon l'une quelconque des revendications 12 à 16, pour déterminer la teneur d'un échantillon en protéine glycosilée, dans lequel la pointe du dispositif de mesure qui, en utilisation, est amenée en contact avec la substance à partir de laquelle un échantillon est à prélever, est de diamètre réduit par rapport au corps du dispositif de mesure.

18. Procédé selon l'une quelconque des revendications 12 à 17, pour déterminer la teneur d'un échantillon en protéine glycosilée, dans lequel le dispositif de mesure comprend un capuchon qui couvre au moins une partie du dispositif de mesure et limite ou empêche les transferts d'échantillon depuis au moins une partie du dispositif de mesure.

19. Procédé selon l'une quelconque des revendications précédentes, pour déterminer la teneur d'un échantillon en protéine glycosilée, dans lequel le dispositif de mesure comprend une mèche.

20. Dispositif pour déterminer la teneur d'un échantillon en protéine glycosilée, par le procédé selon la revendication 1, comprenant un support pour un matériau possédant une affinité pour la protéine glycosilée dont la teneur est à déterminer, des moyens pour amener l'échantillon en contact avec ledit matériau, caractérisé par des moyens pour examiner l'échantillon optiquement, électrochimiquement ou de manière enthapiométrique, et/ou examiner ledit matériau optiquement, électrochimiquement ou par de manière enthalpiométrique avant le contact de l'échantillon avec le matériau et pour examiner l'échantillon optiquement, électrochimiquement ou de manière enthalpiométrique etlou examiner ledit matériau optiquement, électrochimiquement ou de manière enthalpiométrique après le contact de l'échantillon avec le matériau.

21. Dispositif selon la revendication 20, pour déterminer la teneur d'un échantillon en protéine glycosilée, dans lequel ledit matériau comprend de l'acide phénylboronique ou un de ses dérivés.

22. Dispositif selon la revendication 20 ou la revendication 21, pour déterminer la teneur d'un échantillon en protéine glycosilée, dans lequel ledit matériau est lié à un support.

23. Dispositif selon la revendication 22 pour déterminer la teneur d'un échantillon en protéine glycosilée, dans lequel le support comprend au moins un copolymère d'hydroxyéthyl méthacrylate en forme particulaire ou une membrane de nylon.

24. Dispositif selon la revendication 20 ou la revendication 21 pour déterminer la teneur d'un échantillon en protéine glycosilée, dans lequel ledit matériau se présente sous la forme d'un film.

25. Dispositif selon l'une quelconque des revendications 20 à 24 pour déterminer la teneur d'un échantillon en protéine glycosilée, dans lequel l'échantillon est dilué dans une solution tampon avant la mise en présence de l'échantillon et dudit matériau.

26. Dispositif selon l'une quelconque des revendications 20 à 25 pour déterminer la teneur d'un échantillon en protéine glycosilée, dans lequel ledit matériau est maintenu dans un conteneur ou une cartouche.

27. Dispositif selon l'une quelconque des revendications 20 à 26 pour déterminer la teneur d'un échantillon en protéine glycosilée, dans lequel ledit matériau comporte, soit seule soit en combinaison, l'une quelconque des propriétés suivantes qui varient lorsque la protéine glycosilée est liée audit matériau facteur de réflexion, facteur de rétro-réflexion, fluorescence ou phosphorescence.

28. Dispositif selon la revendication 26 pour déterminer la teneur d'un échantillon en protéine glycosilée, dans lequel le conteneur ou la cartouche est immergé dans une certaine quantité de l'échantillon.

29. Dispositif selon la revendication 26 pour déterminer la teneur d'un échantillon en protéine glycosilée, dans lequel on fait passer à force l'échantillon à travers le conteneur ou la cartouche.

**Fig.1**

**Fig.2**

**Fig.3**

**Fig.4**

10